# EUROPÄISCHE PATENTANMELDUNG

(11) **EP 2 226 062 A1**
(43) Veröffentlichungstag der Anmeldung: **08.09.2010**
(21) Anmeldenummer: 09004501.4
(22) Anmeldetag: 27.03.2009
(51) Int. Cl.: A61K 8/04, A61K 8/92, A61Q 19/00, B01J 2/04, B01J 2/06

(54) **Verfahren zur Herstellung von Kosmetika**

(30) Priorität: 06.03.2009 DE 102009012069
(71) Anmelder: Linde AG, 80331 München (DE)
(72) Erfinder: Berghoff, Rudilf Erwin, 85716 Unterschleißheim (DE)
(74) Vertreter: Gellner, Bernd

(57) **Zusammenfassung**

Die Erfindnung betrifft ein Verfahren zur Herstellung eines kosmetischen oder pharmazeutischen Produkts, wobei ein Fett oder Öl in Form eines Granulats (12) mit weiteren Komponenten zu dem Produkt verarbeitet wird.

## Beschreibung

Die Erfindung betrifft ein Verfahren zur Herstellung eines kosmetischen oder pharmazeutischen Produkts, wobei ein Fett, Öl oder Wachs mit weiteren Komponenten zu dem Produkt verarbeitet wird.

Bei der Herstellung von Cremes, Pasten und ähnlichen kosmetischen Produkten werden fettenthaltende Komponenten mit weiteren für das Produkt notwendigen Komponenten, insbesondere Hilfs- und Wirkstoffen, häufig auch mit wässrigen Komponenten, gemischt und verarbeitet. Die wässrigen und die fettigen Komponenten lösen sich nicht oder nur kaum ineinander und bilden daher nach deren Mischung eine Dispersion. Um eine schnelle Entmischung der Dispersion zu verhindern, werden geeignete Emulgatoren und Stabilisatoren eingesetzt.

Aufgabe vorliegender Erfindung ist es, ein Verfahren zur Herstellung von kosmetischen oder pharmazeutischen Produkten aufzuzeigen, welches eine feinere Dispersion der einzelnen Produktkomponenten und höhere Stabilität des Produkts ermöglicht.

Diese Aufgabe wird durch ein Verfahren zur Herstellung eines kosmetischen oder pharmazeutischen Produkts gelöst, wobei ein Fett, Öl oder Wachs mit weiteren Komponenten zu dem Produkt verarbeitet wird, und welches dadurch gekennzeichnet ist, dass das Fett, Öl oder Wachs in Form eines Granulats verarbeitet wird.

Unter dem Begriff "kosmetisches Produkt" sollen alle Arten von pflegenden, reinigenden und dekorativen Kosmetikartikeln verstanden werden. Hierunter fallen beispielsweise Mittel zur Reinigung von Haut, Haar oder Zähnen, wie Reinigungslotionen, Badeöle, Hautcremes, Körperlotionen, Lippenpflegemittel, Shampoos oder Zahnpasten. Aber auch Mittel zur Verschönerung von Haut oder Haar, wie Schminkcremes, Augenpflegemittel, Lippenstifte oder Haargels sind gemeint. Insbesondere soll der Begriff "kosmetisches Produkt" Fett enthaltende Produkte, wie Cremes, Salben, Pasten, Emulsionen und Suspensionen abdecken.

Der Begriff "Granulat" soll insbesondere ein partikelförmiges, pulverförmiges oder körniges Material umfassen.

Der Begriff "Wachs" soll insbesondere tierische und pflanzliche Wachse, auch flüssige Wachse wie zum Beispiel Jojobaöl, erfassen.

Erfindungsgemäß werden Wachs-, Fett- oder Ölpartikel anstatt kohärenter Fettphasen zur Herstellung der kosmetischen Produkte verwendet. Das heißt, das Wachs, Fett oder Öl wird nicht mehr in flüssiger oder zähflüssiger Form mit den weiteren Komponenten verarbeitet, sondern in Form eines Granulats.

In einer bevorzugten Ausführungsform wird das Wachs oder Fett zunächst in den flüssigen Aggregatszustand gebracht. Das Wachs oder Fett wird hierzu entweder geschmolzen oder in einem Lösungsmittel oder in anderen Rezepturbestandteilen gelöst. Anschließend wird die so erhaltene Flüssigkeit versprüht, wobei sich diese in feinste Tröpfchen zerteilt und mit dem umgebenden Gas einen feinen Nebel bildet. Diese kleinen Flüssigkeitströpfchen werden plötzlich abgekühlt, so dass diese ohne zu agglomerieren in den festen Aggregatszustand übergehen und eine granulare bzw. pulverförmige Phase bilden.

Im Falle des Schmelzens erfolgt die Erhitzung aus Gründen der Energieeinsparung vorzugsweise nur bis zu der für den folgenden Sprühprozess notwendigen Minimaltemperatur. Wird die Komponente stattdessen in einem Lösungsmittel gelöst, wird von Vorteil nur die minimal notwendige Lösungsmittelmenge verwendet.

Auf die erfindungsgemäße Weise erhält man ein partikelförmiges, schüttfähiges Material, welches sich schnell und einfach weiter verarbeiten lässt. Das Wachs-, Fett- oder Ölgranulat wird mit den weiteren Komponenten gemischt und gegebenenfalls weiteren Verarbeitungsschritten, wie zum Beispiel einer Erhitzung, unterzogen. Bei Verwendung des erfindungsgemäßen Granulats sind die Einzelkomponenten in dem kosmetischen oder pharmazeutischen Produkt deutlich feindisperser verteilt als bei den herkömmlichen Herstellungsverfahren. Die Homogenität und Stabilität der Produkte wird wesentlich verbessert.

In einer bevorzugten Ausführungsform wird das Fett, Öl oder Wachs im flüssigen Zustand unter erhöhten Druck gesetzt, zu einer Sprühdüse geleitet und versprüht. Es ist ebenso möglich, das Fett, Öl oder Wachs mittels eines Treibgases einer Düse zuzuführen, über die die Komponente abgestrahlt und fein verteilt wird. Das Fett, Öl oder Wachs kann in diesem Fall im Wesentlichen drucklos mit dem Treibgas zusammengeführt werden. Als Treibgas wird vorzugsweise gasförmiger Stickstoff verwendet. Auch andere Zerstäubungs- bzw. Sprühtechniken können eingesetzt werden. Von Vorteil wird über die Art der Sprühtechnik, die Wahl der Sprühdüse und über die Sprühparameter, wie beispielsweise Druck und Geschwindigkeit der flüssigen Komponente unmittelbar vor dem Versprühen, die Tröpfchengröße und damit die Partikelgröße des bei der anschließenden Abkühlung erzeugten Granulats eingestellt.

Das Versprühen des verflüssigten Wachs oder Fetts oder des Öls kann mit Ein- oder Zweistoffdüsen erfolgen. Bei Verwendung von Einstoffdüsen sind je nach gewünschter Tropfen-/Partikelgröße Drücke zwischen 20 und 70 bar, bei sehr kleinen Partikelgrößen auch bis zu 300 bar notwendig. In Zweistoffdüsen kann die Flüssigkeit bei niedrigen Drücken zwischen ca. 2 und 8 bar gefördert werden. Der Druck des Gasstroms, welcher die Zerstäubung bewirkt, liegt üblicherweise um etwa 1 bis 2 bar höher.

Von Vorteil werden das verflüssigte Wachs oder Fett oder das Öl nach dem Versprühen durch Wärmeaustausch mit einem tiefkalten flüssigen Gas, bevorzugt mit flüssigem Stickstoff, abgekühlt. Die Abkühlung kann entweder durch direkten Wärmeaustausch oder durch indirekten Wärmeaustausch erfolgen.

Erfindungsgemäß wird eine öl-, fett- und / oder wachshaltige Komponente des Produkts in ein Granulat überführt. Nach der Verarbeitung der granulierten Öl-, Fett- bzw. Wachskomponenten mit den übrigen Komponenten erhält man ein stabileres Produkt als dies ohne die erfindungsgemäße Verwendung von granulierten Fetten bzw. granulierten Wachsen der Fall ist. Die hergestellten Emulsionen oder Suspensionen zerfallen selbst bei Temperaturschwankungen nicht so schnell in ihre Bestandteile oder agglomerieren, sondern halten den Mischungszustand aufrecht.

Es können aber durchaus auch andere schmelzbare Komponenten vor der Zusammenführung mit den übrigen Komponenten erfindungsgemäß pulverisiert werden. Abhängig von der Rezeptur, nach der das Produkt hergestellt wird, kann es sogar günstig sein, alle Komponenten vor der Vermischung in ein Granulat zu überführen.

Die Erfindung erlaubt es, den Anteil an Emulgatoren und Stabilisatoren im Vergleich zu einem herkömmlich hergestellten Produkt deutlich zu reduzieren. Es ist teilweise sogar möglich, völlig auf Emulgatoren und Stabilisatoren zu verzichten. Auf diese Weise können einerseits die Herstellkosten reduziert werden, andererseits wird ein qualitativ hochwertiges, verbraucherfreundlicheres Produkt erzeugt. Insbesondere Verbraucher, die allergisch auf bestimmte Zusatzstoffe reagieren, profitieren von den erfindungsgemäß hergestellten Produkten.

Nach der Erzeugung des Granulats wird die weitere Verarbeitung mit den übrigen Komponenten von Vorteil bei einer Temperatur von weniger als 25°C, bevorzugt im Temperaturbereich von 0°C bis 15°C, vorgenommen. Im Vergleich zu der herkömmlichen Methode, bei der die Komponenten häufig erhitzt und bei Temperaturen zwischen 40°C und 80°C miteinander gemischt und verarbeitet werden, lassen sich dadurch deutliche Energieeinsparungen erzielen. Je nach dem, welche Komponenten zu dem Produkt verarbeitet werden, können sogar Temperaturen von weniger als 0°C wünschenswert sein.

Die Erfindung beruht wesentlich auf der Umwandlung mindestens einer öl-, fett- und/oder wachshaltigen Komponente in einen partikelförmigen, granularen oder pulverförmigen Zustand. In diesem Zustand lässt sich die Komponente besser mit den anderen Komponenten mischen und man erhält eine deutlich feinere Dispersion. Besonders schwermischbare Produkte, wie Wasser-Öl-Komponenten lassen sich wesentlich einfacher in Emulsionen überführen, ohne dass die einzelnen Phasen zu größeren Bereichen zusammenwachsen.

Vorzugsweise wird das Fett, Öl oder Wachs in ein Granulat überführt, welches im Wesentlichen Teilchengrößen zwischen 1 Mikrometer und 1000 Mikrometer, bevorzugt zwischen 1 Mikrometer und 300 Mikrometer, aufweist. Die Komponente wird hierzu von Vorteil im flüssigen Aggregatszustand so fein zerstäubt bzw. versprüht, dass sich entsprechende kleine Tröpfchen bilden, die anschließend schockgekühlt und in den festen Aggregatszustand überführt werden.

Die Verwendung dieses feinen Wachs-, Öl- oder Fett-Granulats erleichtert die Herstellung der gewünschten Emulsionen oder Suspensionen, aus denen das Produkt besteht. Die Verteilung des Fetts, Öls oder Wachs in den übrigen Komponenten ist deutlich homogener als bei den üblichen Herstellungsverfahren, bei denen das Fett, Öl oder Wachs im flüssigen Zustand mit den anderen Komponenten gemischt wird.

Die Erfindung hat zahlreiche Vorteile im Vergleich zu den bekannten Herstellungsverfahren für kosmetische oder pharmazeutische Produkte. Das feine Granulat weist sehr gleichmäßige Partikelgrößen auf und wird dadurch sehr homogen mit den anderen Komponenten vermischt, so dass das Endprodukt eine deutlich höhere Stabilität aufweist. Das erfindungsgemäße Granulat ist schüttfähig, leicht dosierbar und somit einfacher, schneller und billiger weiterzuverarbeiten. Durch die feine Dispersion der Einzelkomponenten im Produkt wird es möglich, den Anteil an Emulgatoren und Stabilisatoren zu reduzieren, teilweise sogar gänzlich auf diese zu verzichten. So hergestellte Produkte besitzen eine höhere Qualität, beispielsweise sind Pflegeprodukte hautfreundlicher, und zudem kostengünstiger in der Herstellung.

Die Erfindung bringt in der kosmetischen Industrie bei der Herstellung von Emulsionen und Suspensionen aller Art Vorteile. Einige bevorzugte Anwendungsgebiete sind die Herstellung von
- Silan- oder Silikon-Emulsionen: Diese finden in der Kosmetik in Form eines Gels oder als Emulsion insbesondere Verwendung in Hautcremes und Sonnenschutzprodukten.
- Gel: Gele sind formbeständige, leicht deformierbare, feindisperse Systeme aus mindestens einer festen und einer wässrigen Phase. Gele werden beispielsweise als Trägermedium für Salben und Cremes oder als Haargel verwendet.
- Öl-Wasser-Emulsionen, wie sie zum Beispiel in sprühbaren Sonnenschutzmitteln eingesetzt werden.
- Shampoo und Haarpflegemittel
- Cremes und Salben mit einem hohen Anteil an Fetten und/oder Wachsen

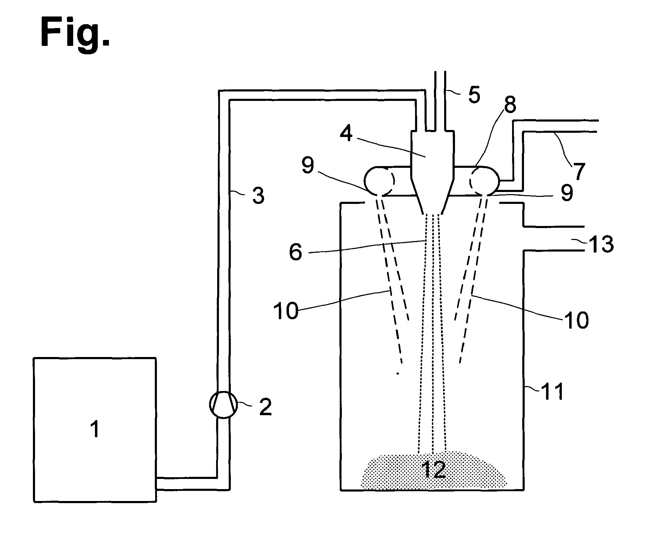

Die Erfindung sowie weitere Einzelheiten der Erfindung werden im Folgenden anhand von dem in der Zeichnung dargestellten Ausführungsbeispiel näher erläutert. Hierbei zeigt die

Figur eine Vorrichtung zur Durchführung des erfindungsgemäßen Verfahrens.

Die Figur zeigt eine Vorrichtung zur Herstellung von feinen Öl- oder Fettpartikeln, die anschließend mit weiteren Komponenten zu Kosmetika verarbeitet werden. Das flüssige Fett oder das Öl werden in einem Speicherbehälter 1 vorgehalten. Bei der Verwendung von Fett wird dieses zuvor bis knapp über dessen Schmelzpunkt erhitzt und dadurch verflüssigt.

Das Öl oder Fett wird mittels einer Pumpe 2 bei einem Druck zwischen 1,5 und 8 bar über eine Leitung 3 zu einer Zweistoffdüse 4 gefördert. Der Zweistoffdüse 4 wird über Leitung 5 außerdem gasförmiger Stickstoff als Zerstäubergas zugeführt. In der Düse 4 wird das flüssige Fett oder Öl mit dem Zerstäubungsgas gemischt und als Zwei-Phasen-Strömung in einen Produktbehälter 11 gestrahlt. Das flüssige Fett oder Öl liegt in diesem Zwei-Phasen-Gemisch als fein verteilte Tröpfchen 6, vorzugsweise mit einer Tröpfchengröße zwischen 10 und 500 Mikrometer, vor. Die Tröpfchengröße kann durch Variation des Drucks des Zerstäubungsgases und/oder des Durchsatzes an flüssigem Fett oder Öl gesteuert werden.

Die Abkühlung der Fett- oder Öltröpfchen erfolgt durch Wärmeaustausch mit kryogenem Stickstoff. Hierzu wird flüssiger Stickstoff über Leitung 7 einer Ringleitung 8 zugeführt. Die Ringleitung 8 ist konzentrisch um die Zweistoffdüse 4 angeordnet und besitzt an ihrer Unterseite mehrere Austrittsöffnungen 9, aus denen feine Strahlen 10 flüssigen Stickstoffs ausgestoßen werden. Die Strahlen 10 kryogenen Stickstoffs sind schräg nach unten und innen ausgerichtet und treten mit den Fett- oder Öltröpfchen 6 in Wärmeaustausch, wobei sich die Fett- oder Öltröpfchen 6 zu kleinen Partikeln 12 verfestigen und am Boden des Produktbehälters 11 sammeln. Der als Kühlmedium eingesetzte flüssige Stickstoff verdampft und wird zusammen mit dem Zerstäubungsgas über einen Abzug 13 abgesaugt.

Die erzeugten festen Fett- oder Ölpartikel werden dann als Ausgangsstoff für die Herstellung von Kosmetika verwendet.

## Patentansprüche

1. Verfahren zur Herstellung eines kosmetischen oder pharmazeutischen Produkts, wobei ein Fett, Öl oder Wachs mit weiteren Komponenten zu dem Produkt verarbeitet wird, **dadurch gekennzeichnet, dass** das Fett, Öl oder Wachs in Form eines Granulats (12) verarbeitet wird.

2. Verfahren nach Anspruch 1, **dadurch gekennzeichnet, dass** das Fett, Öl oder Wachs im flüssigen Zustand versprüht (6), schockartig abgekühlt und in ein Granulat (12) überführt wird.

3. Verfahren nach Anspruch 2, **dadurch gekennzeichnet, dass** das Fett, Öl oder Wachs durch Wärmeaustausch, insbesondere direkten Wärmeaustausch, mit einem tiefkalten flüssigen Gas (10), bevorzugt mit flüssigem Stickstoff, abgekühlt wird.

4. Verfahren nach einem der Ansprüche 1 bis 3, **dadurch gekennzeichnet, dass** das Granulat (12) mit den weiteren Komponenten bei einer Temperatur von weniger als 25°C, bevorzugt im Temperaturbereich von 0°C bis 15°C, verarbeitet wird.

5. Verfahren nach Anspruch 4, **dadurch gekennzeichnet, dass** das Granulat (12) mit den weiteren Komponenten bei einer Temperatur von weniger als 0°C verarbeitet wird.

6. Verfahren nach einem der Ansprüche 1 bis 5, **dadurch gekennzeichnet, dass** das Granulat (12) eine Teilchengröße zwischen 1 und 1000 Mikrometer besitzt.

7. Verfahren nach einem der Ansprüche 1 bis 6, **dadurch gekennzeichnet, dass** das Granulat (12) mit den weiteren Komponenten ohne Zusatz von Emulgatoren oder Stabilisatoren verarbeitet wird.

8. Verfahren nach einem der Ansprüche 1 bis 7, **dadurch gekennzeichnet, dass** eine Creme oder Paste hergestellt wird.
